# FASCICULE DE BREVET EUROPEEN

(11) **EP 3 291 791 B1**
(45) Date de publication et mention de la délivrance du brevet: **22.07.2020**
(21) Numéro de dépôt: 16719877.9
(22) Date de dépôt: 03.05.2016
(51) Int. Cl.: A61K 8/31, A61K 9/107, A61K 8/06, A61Q 19/00, A61K 47/06

(54) **NANO-EMULSION COSMETIQUE**
KOSMETISCHE NANOEMULSION
COSMETIC NANOEMULSION

(30) Priorité: 04.05.2015 FR 1553980
(43) Date de publication de la demande: 14.03.2018
(73) Titulaire: Total Marketing Services, 92800 Puteaux (FR)
(72) Inventeur: RODRIGUEZ, Corinne, 60000 Beauvais (FR); MAGNIEZ, Harmonie, 60840 Nointel (FR); COLETTA, Daniel, 60310 La Trinité de Thouberville (FR); QUILLET, Serge, 78220 Viroflay (FR); SWOBODA, Benjamin, 78630 Orgeval (FR)
(74) Mandataire: Corizzi, Valérie
(86) Numéro de dépôt international: PCT/EP2016/059849
(87) Numéro de publication internationale: WO 2016/177704

(56) Documents cités:
- WO-A1-02/32390
- WO-A1-2010/085753
- WO-A1-2012/085491
- WO-A2-2007/095255
- US-A1- 2007 241 306
- US-A1- 2009 176 876

## Description

### DOMAINE DE L'INVENTION

L'invention concerne une composition cosmétique ou dermatologique sous forme d'une nano-émulsion huile-dans-l'eau obtenue par la technique de température d'inversion de phase.

L'invention concerne également les utilisations de ladite composition.

### CONTEXTE TECHNIQUE DE L'INVENTION

Il est commun d'utiliser dans les domaines cosmétique et dermatologique des émulsions huile-dans-eau (H/E). Ces émulsions qui sont constituées d'une phase lipophile (encore appelée phase huileuse ou phase grasse) dispersée dans une phase aqueuse ont, de ce fait, une phase aqueuse externe et sont donc des produits agréables à utiliser, en raison de la sensation de fraîcheur qu'ils procurent. La fabrication d'émulsions directes concentrées en huile est souvent difficile et les émulsions obtenues sont souvent instables.

Ces émulsions sont classiquement obtenues par voie mécanique, par exemple par émulsification avec un rotor stator ou à l'aide d'un homogénéisateur haute-pression, un apport d'énergie important étant nécessaire pour diviser la phase dispersée en petites gouttes. Pour stabiliser ces émulsions, des émulsionnants non-ioniques du type huile-dans-l'eau (H/E), c'est-à-dire de HLB (Hydrophilic Lipophilic Balance) allant de 8 à 18 sont généralement ajoutés. Ces émulsionnants, grâce à leur structure amphiphile, se placent à l'interface phase huile/phase aqueuse, et stabilisent ainsi les gouttelettes d'huile dispersées. Malgré la présence d'émulsionnants, les émulsions peuvent avoir tendance à se déstabiliser. On assiste alors à un phénomène de coalescence puis séparation des phases aqueuse et huileuse avec relargage d'huile. Pour améliorer la stabilité de ces nano-émulsions, on peut augmenter la concentration en émulsionnants mais à de fortes concentrations, ils entraînent un toucher rêche, collant ou poisseux ainsi que des problèmes d'innocuité vis-à-vis de la peau, des yeux ou des muqueuses.

Pour résoudre les problèmes de stabilité des émulsions directes classiques, il a été proposé de réaliser des émulsions directes obtenues par la technique de température d'inversion de phase (émulsions PIT). Ces émulsions PIT encore appelées nano-émulsions ont une taille moyenne de globules constituant la phase lipophile comprise dans des limites déterminées, c'est-à-dire inférieure au nanomètre. Le principe d'émulsification par la technique de température d'inversion de phase (en anglais : Phase Inversion Temperature ou PIT) est, dans son principe, bien connu de l'homme du métier. Il a été décrit en 1968 par K. Shinoda (J. Chem. Soc. Jpn, 1968, 89, 435). Il a été montré que cette technique permet d'obtenir des émulsions fines et stables (K. Shinoda et H. Saito, J. Colloïd Interface Sci., 1969, 30, 258). Cette technologie a été appliquée en cosmétique dès 1972 par Mitsui et al. (« Application of the phase-inversion-temperature method to the emulsification of cosmetics"; T. Mitsui, Y. Machida and F. Harusawa, American, Cosmet. Perfum, 1972, 87, 33).

Par ailleurs, il est avantageux de disposer d'émulsions fines, c'est-à-dire d'émulsions où la phase lipophile se trouve sous forme de nano gouttelettes, c'est-à-dire de taille inférieure au nanomètre, car ces émulsions très fines sont généralement plus stables que les émulsions directes obtenues par voie mécanique. Ces émulsions, encore appelées nano-émulsions, ont des reflets bleutés et un comportement newtonien. Ces nano-émulsions sont très utilisées pour les produits vaporisables, dans des domaines variés tels que le soin, les anti-transpirants, le solaire ou notamment dans les lingettes.

Le principe de cette technique est le suivant : on réalise le mélange d'une phase aqueuse et d'une phase lipophile, que l'on porte à une température supérieure à la température de PIT, température d'inversion de phase du système, qui est la température à laquelle l'équilibre entre les propriétés hydrophiles et lipophiles du ou des émulsionnant(s) mis en œuvre est atteint. A température élevée c'est-à-dire supérieure à la température d'inversion de phase (>T_{PIT}), l'émulsion est de type eau-dans-l'huile. Au cours de son refroidissement, elle passe par un état de microémulsion ou nano émulsion. L'émulsion eau-dans-l'huile s'inverse donc à la température d'inversion de phase, pour devenir une émulsion de type huile-dans-l'eau. L'avantage du procédé est de fournir des émulsions plus fines que les émulsions classiques.

Les phases lipophiles, ou encore appelées phases grasses ou phases huileuses, de ces nano-émulsions sont généralement constituées d'alcanes linéaires (par exemple les nonane, dodécane ou tétradécane), des huiles minérales ou encore des isoparaffines. Dans le cas des alcanes linéaires, la formation de la nano-émulsion est influencée par la longueur de chaine carbonée : plus la chaine carbonée est courte, plus la température de PIT diminue. Cependant les formulations sont limitées car pour un nombre de carbones inférieur à 12 (C<12) les n-alcanes sont irritants et donc peu utilisés en cométique. Et au dessus de 14 carbones (C>14), le point d'écoulement (noté PP pour « Pour Point » en Anglais) est supérieur à 5°C, donc les émulsions ne sont pas utilisables « à froid » ou vaporisables. («The effect of combination of various oils, emulsifiers and additives on the phase inversion temperature in emulsion systems» Bulletin of the Chemical Society of Japan (1970), 43(10), 3044-8, Mitsui, Takeo; Machida, Yasuhiko; Harusawa, Fuminori ; «Assessment of skin irritation and molecular responses in rat skin exposed to nonane, dodecane, tetradecane», RJ Babu, 2004). Les huiles minérales (paraffinum liquidum en cosmétique) sont utilisées pour ce type de composition, cependant leur forte viscosité engendre des températures de PIT élevées et donc des consommations énergétiques importantes lors de la réalisation des émulsions. De plus, les huiles minérales sont connues pour être occlusives pour la peau et irritantes si elles ne sont pas assez pures. Les nano-émulsions comprenant uniquement des isoparaffines en tant que phase grasse ne sont pas stables. En effet le taux de branchement des molécules déstabilise l'émulsion. Par exemple l'isohexadécane, très utilisé en cosmétique, ne peut être utilisé seul pour formuler des émulsions PIT en raison de la faible stabilité des émulsions obtenues. De plus pour C<12 et à des concentrations importantes, ce qui est le cas des nano émulsions avec des concentrations en phase grasse autour de 20%, les isoparaffines sont irritantes car elles pénètrent dans la peau. (« Formation and stability of nano-emulsions » Advances in Colloid and Interface Science (2004), 108-109, 303-318, Tadros, Tharwat; Izquierdo, P.; Esquena, J.; Solans, C.). Le document WO 02/032390 A1 décrit des compositions parfumantes sous forme de nano-émulsion huile dans eau. Les exemples de ce document utilisent des tensioactifs non-ioniques polyéthoxylés qui appartiennent aux familles suivantes : les stéaryléthers de polyéthylèneglycol (Brij® 78P et Brij® 721) et les oleyléthers de polyéthylèneglycol (Brij® 98V).

Enfin, le problème de stabilité des émulsions PIT dû à certaines phases grasses peut éventuellement être surmonté en mettant en œuvre une quantité de système émulsionnant importante par rapport à la quantité de phase lipophile. Mais on aboutit alors à une composition présentant des qualités cosmétiques peu satisfaisantes, notamment en termes de toucher, de soin de la peau, notamment de protection de la peau contre la déshydratation.

Il subsiste donc encore le besoin d'améliorer les caractéristiques intrinsèques de ces nano-émulsions PIT.

La demanderesse a trouvé de manière surprenante que le choix de composés particuliers dans la phase lipophile permet de réaliser des nano-émulsions PIT stables et non irritantes avec une température d'inversion de phase faible.

La demanderesse a découvert de manière surprenante que le choix d'un système émulsionnant particulier permet de réaliser des nano-émulsions PIT stables, non irritantes, avec une température d'inversion de phase faible et avec une quantité de phase lipophile élevée.

La présente invention a pour objectif de fournir une composition cosmétique ou dermatologique obtenue à faible température d'inversion de phase.

L'invention a également pour objectif de proposer une composition cosmétique ou dermatologique stable.

### RESUME DE L'INVENTION

Ces objectifs sont atteints grâce à une nouvelle composition cosmétique ou dermatologique.

L'invention concerne une composition cosmétique ou dermatologique sous forme d'une nano-émulsion huile-dans-eau comprenant :
- une phase lipophile (a) qui comprend au moins une huile hydrocarbonée comprenant au moins une isoparaffine, et au moins un cyclo-alcane,
- un système émulsionnant (b) comprenant au moins 95% en poids d'émulsionnants choisis parmi les produits d'addition d'oxyde d'éthylène avec l'alcool cétéarylique ou cétylstéarylique comportant de 10 à 20 groupes oxyéthylénés, du Stéarate de glycéryle et de l'alcool cétéarylique, par rapport au poids total du système émulsionnant,
- une phase aqueuse (c),
- le rapport en poids phase lipophile (a) / système émulsionnant (b) étant supérieur ou égal à 0,5.

De préférence, le système émulsionnant de la composition selon l'invention est composé de au moins 95%, plus préférentiellement au moins 98% voir au moins 99% en poids, d'émulsionnants choisis parmi les produits d'addition d'oxyde d'éthylène avec l'alcool cétéarylique ou cétylstéarylique comportant de 10 à 20 groupes oxyéthylénés, du Stéarate de glycéryle et de l'alcool cétéarylique, par rapport au poids total du système émulsionnant.

De préférence, le système émulsionnant de la composition selon l'invention est constitué du ceteareth-20, du ceteareth-12, de l'alcool cétéarylique et du stéarate de glycéryle.

De préférence, la composition de l'invention comprend une quantité de phase lipophile (a) allant de 5% à 40%, préférentiellement de 10% à 35%, plus préférentiellement de 15% à 30% et encore plus préférentiellement de 20% à 30% en poids par rapport au poids total de la composition.

De préférence, la phase lipophile (a) de la composition de l'invention comprend de 15% à 100% en poids d'huile hydrocarbonée, préférentiellement de 20% à 90%, plus préférentiellement de 25% à 80% et encore plus préférentiellement entre de 30% à 70% en poids d'huile hydrocarbonée par rapport au poids total de la phase lipophile.

De préférence, l'huile hydrocarbonée de la phase lipophile (a) de la composition de l'invention est une huile hydrocarbonée hydrogénée.

De préférence, l'huile hydrocarbonée de la phase lipophile (a) de la composition de l'invention est choisie parmi les isoparaffines, les paraffines normales et/ou les cyclo alcanes comprenant 12 à 29 atomes de carbone, préférentiellement 13 à 23 atomes de carbone et plus préférentiellement 18 à 21 atomes de carbone.

De préférence, l'huile hydrocarbonée de la phase lipophile (a) de la composition de l'invention a une teneur en composés paraffiniques allant de 40% à 99%, préférentiellement de 45% à 90% et plus préférentiellement de 60% à 80%.

De préférence, l'huile hydrocarbonée de la phase lipophile (a) de la composition de l'invention a une teneur en composés naphténiques allant de 1% à 60%, préférentiellement de 10% à 55 % et plus préférentiellement de 20% à 40 %.

De préférence, l'huile hydrocarbonée de la phase lipophile (a) de la composition de l'invention a une teneur en isoparaffines allant de 30% à 90%, de préférence de 40% à 80% et plus préférentiellement de 50% à 70%.

De préférence, l'huile hydrocarbonée de la phase lipophile (a) de la composition de l'invention a une teneur en paraffines normales allant de 0,01% à 30%, de préférence de 0,01% à 25% et plus préférentiellement de 5% à 20%.

Avantageusement, l'huile hydrocarbonée de la phase lipophile (a) de la composition de l'invention comprend de 30% à 90% d'isoparaffines, de 0,01% à 30% de paraffines normales et de 1% à 60% de composés naphténiques.

De préférence, la composition de l'invention comprend une quantité de système émulsionnant (b) allant de 2% à 15%, préférentiellement de 3% à 14%, plus préférentiellement de 4% à 12% et encore plus préférentiellement de 5% à 11% en poids par rapport au poids total de la composition.

Avantageusement, le système émulsionnant (b) de la composition de l'invention comprend au moins un émulsionnant lipophile et un émulsionnant hydrophile.

Selon un mode de réalisation, la composition de l'invention est obtenue par la technique de température d'inversion de phase.

Un autre objet de l'invention est l'utilisation cosmétique de la composition de l'invention pour une application topique. Elle concerne en particulier son utilisation comme produit de soin solaire, comme produit de soin de la peau ou comme produit de maquillage.

Un autre objet de l'invention est aussi l'utilisation de la composition de l'invention pour la formulation de produits cosmétiques ou de produits dermatologiques.

### DESCRIPTION DETAILLEE DE L'INVENTION

L'invention concerne une composition cosmétique ou dermatologique sous forme d'une nano-émulsion huile-dans-eau comprenant une phase lipophile qui comprend au moins une huile hydrocarbonée comprenant au moins une isoparaffine et au moins un cyclo-alcane, éventuellement au moins une paraffine normale, un système émulsionnant comprenant au moins un émulsionnant non ionique et une phase aqueuse avec un rapport phase lipophile / système émulsionnant supérieur ou égal à 0,5 en poids.

L'invention concerne en particulier une composition comprenant une nano-émulsion huile-dans-l'eau (H/E) obtenue par la technique de température d'inversion de phase (PIT).

La composition selon l'invention comprend en particulier une nano-émulsion H/E permettant d'obtenir une composition stable.

La composition selon l'invention comprend en particulier une nano-émulsion H/E permettant une inversion de phase à une faible température.

La composition selon l'invention comprend en particulier une nano-émulsion H/E dont la phase lipophile est non-irritante, non-occlusive et biodégradable.

### Phase lipophile :

La composition selon l'invention comprend de préférence une quantité de phase lipophile, appelée également phase grasse ou phase huileuse, allant de 5% à 40%, préférentiellement de 10% à 35%, plus préférentiellement de 15% à 30 % et encore plus préférentiellement de 20% à 30% en poids par rapport au poids total de la composition.

La présence d'une quantité significative de phase lipophile contribue aux qualités cosmétiques de la composition : toucher agréable, soin et protection de la peau, notamment contre la déshydratation.

La phase lipophile de la composition selon l'invention comprend au moins une huile hydrocarbonée. L'huile hydrocarbonée a de préférence une teneur en composés paraffiniques allant de 40% à 99%, préférentiellement de 45% à 90% et plus préférentiellement de 60% à 80%. En règle générale ces paraffines sont des mélanges.

L'huile hydrocarbonée comprend avantageusement un mélange d'isoparaffines et de paraffines normales. De préférence la teneur en isoparaffines de l'huile hydrocarbonée est de 30% à 90 %, préférentiellement de 40% à 80% et plus préférentiellement de 50% à 70 %. De préférence la teneur en paraffines normales de l'huile hydrocarbonée est de 0.01% à 30%, préférentiellement de 0.01% à 25 % et plus préférentiellement de 5% à 20%.

L'huile hydrocarbonée a également de préférence une teneur en composés naphténiques, encore appelés cyclo-alcanes, allant de 1% à 60 %, préférentiellement de 10% à 55 % et plus préférentiellement de 20% à 40 %.

Selon un mode de réalisation préféré, l'huile hydrocarbonée comprend de 30% à 90% d'isoparaffines, de 0,01% à 30% de paraffines normales et de 1% à 60% de cyclo-alcanes. De préférence l'huile hydrocarbonée comprend de 40% à 80% d'isoparaffines, de 0,01% à 25% de paraffines normales et de 10 à 55% de cyclo-alcanes. Plus préférentiellement, l'huile hydrocarbonée comprend de 50% à 70% d'isoparaffines, de 5% à 20% de paraffines normales et de 20% à 40% de cyclo-alcanes.

L'huile hydrocarbonée mise en œuvre dans la composition selon l'invention est avantageusement exempte de composés aromatiques. Par exempt, on entend une teneur en composés aromatiques inférieure à 500 ppm, de préférence inférieure à 300 ppm et plus préférentiellement inférieure à 150 ppm mesurée par spectrométrie UV.

L'huile hydrocarbonée a également de préférence une viscosité cinématique à 40°C allant de 1 à 12 Cst, préférentiellement de 2 à 8 Cst et plus préférentiellement de 3 à 7 Cst selon la norme ASTM D445.

L'huile hydrocarbonée a de préférence un point d'écoulement typique selon la norme ASTM D97 allant de 10 à -60°C, préférentiellement de 5 à -50°C et plus préférentiellement de 0 à -20 °C.

De telles compositions d'huiles hydrocarbonées peuvent être obtenues de la façon suivante. L'huile hydrocarbonée selon l'invention est une coupe hydrocarbonée qui peut être issue de façon connue du pétrole brut ou de la biomasse.

De préférence, on entend par coupe hydrocarbonée au sens de l'invention, une coupe issue de la distillation du pétrole brut, de préférence issue de la distillation atmosphérique et/ou distillation sous vide du pétrole brut, de préférence issue de la distillation atmosphérique suivie de la distillation sous vide.

La coupe hydrocarbonée mise en œuvre dans la composition cosmétique ou dermatologique de l'invention est avantageusement obtenue par un procédé comprenant des étapes d'hydrotraitement, hydrocraquage ou craquage catalytique.

La coupe hydrocarbonée mise en œuvre dans la composition cosmétique ou dermatologique de l'invention est de préférence obtenue par un procédé comprenant des étapes de désaromatisation et éventuellement de désulfuration.

De préférence, la coupe hydrocarbonée obtenue après la ou les étapes de distillation est une coupe gazole. Cette coupe gazole est de préférence obtenue par un procédé comprenant des étapes d'hydrotraitement, d'hydrocraquage, de craquage catalytique, suivi éventuellement des étapes de désaromatisation et éventuellement de désulfuration.

La coupe hydrocarbonée peut être un mélange de coupes hydrocarbonées ayant subi les étapes décrites ci-dessus.

La coupe hydrocarbonée mise en oeuvre dans la composition de l'invention peut également être issue de la conversion de la biomasse.

Par issue de la conversion de la biomasse, on entend une coupe hydrocarbonée produite à partir de matières premières d'origine biologique choisies de préférence parmi les huiles végétales, les graisses animales, les huiles de poisson et leurs mélanges. Les matières premières d'origine biologique appropriées sont par exemple l'huile de colza, l'huile de canola, le talloil, l'huile de tournesol, l'huile de soja, l'huile de chanvre, l'huile d'olive, l'huile de lin, l'huile de moutarde, l'huile de palme, l'huile d'arachide, l'huile de ricin, l'huile de noix de coco, les graisses animales telles que le suif, les graisses alimentaires recyclées, les matières premières issues du génie génétique, et les matières premières biologiques produites à partir de microorganismes tels que les algues et les bactéries.

De préférence, La coupe hydrocarbonée d'origine biologique est obtenue par un procédé comprenant des étapes d'hydrodésoxygénation (HDO) et d'isomérisation. L'étape d'hydrodésoxygénation (HDO) conduit à la décomposition des structures des esters biologiques ou des constituants triglycérides, à l'élimination des composés oxygénés, phosphorés et soufrés et à l'hydrogénation des liaisons oléfiniques. Le produit issu de la réaction d'hydrodésoxygénation est ensuite isomérisé. Une étape de fractionnement peut de préférence suivre les étapes d'hydrodésoxygénation et d'isomérisation.

Les fractions d'intérêt sont ensuite soumises à des étapes d'hydrotraitement puis de distillation afin obtenir les spécifications de la coupe hydrocarbonée souhaitée selon l'invention.

La coupe hydrocarbonée peut être un mélange de coupe hydrocarbonée issue de la distillation du pétrole brut et / ou de la conversion de la biomasse.

L'huile hydrocarbonée mise en œuvre dans la composition de l'invention est avantageusement une coupe hydrocarbonée ayant un intervalle de distillation ID (en °C) allant de 220°C à 380°C, de préférence de 220°C à 370°C et encore plus préférentiellement de 240°C à 335°C mesuré selon la norme ASTM D86. De préférence, la différence entre le point d'ébullition initial et le point d'ébullition final est inférieure ou égale à 150°C. L'huile hydrocarbonée peut comprendre une ou plusieurs fractions d'intervalles de distillation compris dans les intervalles décrits ci-dessus.

Avantageusement, l'huile hydrocarbonée mise en œuvre dans la composition cosmétique ou dermatologique de l'invention est totalement saturée. De préférence, les composants de l'huile hydrocarbonée sont choisis parmi les isoparaffines, les paraffines normales et les cyclo-alcanes comprenant 12 à 29 atomes de carbone, préférentiellement 13 à 23 atomes de carbone et plus préférentiellement 18 à 21 atomes de carbones.

Avantageusement ladite phase lipophile de la composition cosmétique ou dermatologique selon l'invention comprend de 15% à 100 % en poids d'au moins une huile hydrocarbonée telle que décrite ci-dessus, préférentiellement de 20% à 90%, plus préférentiellement de 25% à 80% et encore plus préférentiellement de 30% à 70% en poids par rapport au poids total de la phase lipophile.

La phase lipophile peut en outre contenir jusqu'à 85% en poids par rapport au poids total de la phase lipophile, d'une ou plusieurs autres huiles choisies parmi les esters d'acides gras, les éthers, les huiles végétales, les huiles de silicone, les huiles fluorées, volatiles ou non. On peut notamment citer comme huiles de ce type :
- les esters, tels que le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le monococoate d'éthyl-2 hexyle (ou monococoate d'octyle), le palmitate de méthyle, le palmitate d'éthyle, le palmitate d'isopropyle, le palmitate d'isobutyle, le stéarate de butyle, le stéarate d'isopropyle, le stéarate d'isobutyle, , l'isostéarate d'isopropyle, le pelargonate d'éthyl-2 hexyle (ou pelargonate d'octyle), l'hydroxystéarate d'éthyl-2 hexyle (ou hydroxystéarate d'octyle), le décyl oléate, l'adipate de di-isopropyle, l'adipate de di-éthyl-2 hexyle (ou adipate de dioctyle), l'adipate de diisocétyle, le succinate d'éthyl-2 hexyle (ou succinate d'octyle), le sébacate de diisopropyle, le malate d'éthyl-2 hexyle (ou malate d'octyle), le caprate/caprylate de pentaérythritol,
   l'hexanoate d'éthyl-2 hexyle (ou hexanoate d'octyle), l'octanoate d'octyldodécyle, le néopentanoate d'isodécyle, le néopentanoate d'isostéaryle, l'isononanoate d'isononyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle, le lauroyl sarcosinate d'isopropyle (Eldew SL 205 de Unipex), le dicaprylyl carbonate (Cetiol CC de Cognis), et leurs mélanges ;
- les éthers tels que le Dicaprylyl éther (Cetiol OE de Cognis) ;
- les huiles hydrocarbonées d'origine végétale telles que l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;
- les huiles de silicone, volatiles ou non, comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ;
- les huiles fluorées telles que celles partiellement hydrocarbonées et/ou siliconées comme celles décrites dans le document JP-A-2-295912.

Les autres constituants lipophiles pouvant être présents dans la phase lipophile sont par exemple les cires ; les gommes telles que les gommes de silicone (diméthiconol) ; les résines de silicone telles que la trifluorométhyl-C1-4-alkyldimethicone et la trifluoropropyldimethicone ; les pâtes telles que Petrolatum ; et leurs mélanges.

### Système émulsionnant :

Les systèmes d'émulsionnants non-ioniques comprenant au moins un émulsionnant hydrophile et au moins un émulsionnant lipophile permettent de réaliser des émulsions par la technique de température d'inversion de phase appelées émulsions PIT réputées très stables. Le calcul d'inversion de phase dans les émulsions concentrées (CAPICO en anglais) est possible à partir des valeurs EACN (nombre de carbones équivalent en alcane) et donne une proportion d'émulsionnants et de phase grasse correspondant à une température donnée d'inversion de phase (PIT). En outre, CAPICO est une méthode de calcul, déterminant la dose d'émulsionnant nécessaire pour émulsionner une phase grasse imposée (construite par rapport aux caractéristiques sensorielles et chimiques) (International Journal of Cosmetic Science 1994 apr ; 16(2) : 84-92 « Calculation of optimum emulsifier mixtures for phase inversion emulsification » Förster T, Rybinski WV, Tesmann H, Wadle A (Henkel KGaA)).

Les émulsionnants présentent la particularité de faciliter la dispersion de deux phases insolubles l'une dans l'autre. La HLB (Hydrophilic Lipophilic balance) est le rapport entre la partie hydrophile et la partie lipophile dans leur molécule. Ce terme HLB est bien connu de l'homme du métier et est décrit dans "The HLB system. A time-saving guide to Emulsifier Selection" (publié par ICI Americas Inc; 1984).

Le système émulsionnant utilisé dans la composition selon l'invention comprend un ou plusieurs émulsionnant(s) non-ionique(s). De préférence, il comprend au moins un émulsionnant hydrophile et au moins un émulsionnant lipophile.

De préférence, ces émulsionnants non-ioniques sont des émulsionnants oxyéthyléné(s) et/ou glycérolé(s) de HLB allant de 8 à 18, et de préférence de 10 à 16.

Le système émulsionnant peut comprendre, en particulier, un ou plusieurs émulsionnant(s) non-ionique(s) oxyéthyléné(s) et/ou glycérolé(s) choisi(s) parmi les alcools gras éthoxylés, les esters d'acides gras et de PEG, les glycérides partiels d'acides gras éthoxylés, les triglycérides d'acides gras polyglycérolés et leurs dérivés éthoxylés.

Les alcools gras éthoxylés sont de préférence choisis parmi les alcools gras en C18 à C22 et possédant de 6 à 12, et de préférence de 8 à 12 moles d'oxyde d'éthylène. Comme alcools gras éthoxylés, on peut notamment citer par exemple les produits d'addition d'oxyde d'éthylène avec l'alcool béhénylique, notamment ceux comportant de 6 à 12 groupes oxyéthylénés (par exemple Beheneth-9 ou Beheneth-10 selon la dénomination du CTFA) ; les produits d'addition d'oxyde d'éthylène avec l'alcool stéarylique, notamment ceux comportant de 2 à 21 groupes oxyéthylénés (par exemple Steareth-9 selon la dénomination du CTFA) ; les produits d'addition d'oxyde d'éthylène avec l'alcool isostéarylique, notamment ceux comportant de 6 à 12 groupes oxyéthylénés (Isosteareth-9 selon la dénomination du CTFA) ; et leurs mélanges.

A titre d'émulsionnants non-ioniques oxyéthylénés et/ou glycérolés de HLB allant de 8 à 18, la composition peut également comprendre des alcools gras oxyéthylénés différents de ceux décrits ci-dessus à savoir, les produits d'addition d'oxyde d'éthylène avec l'alcool laurylique, notamment ceux comportant de 9 à 50 groupes oxyéthylénés (Laureth-9 à Laureth-50 selon la dénomination CTFA) ; les produits d'addition d'oxyde d'éthylène avec l'alcool cétéarylique ou cétylstéarylique (mélange d'alcool cétylique et d'alcool stéarylique), notamment ceux comportant de 9 à 30 groupes oxyéthylénés (Ceteareth-9 à Ceteareth-30 selon la dénomination CTFA) ; les produits d'addition d'oxyde d'éthylène avec l'alcool cétylique, notamment ceux comportant de 9 à 30 groupes oxyéthylénés (Ceteth-9 à Ceteth-30 selon la dénomination CTFA) ; et leurs mélanges.

Les esters d'acide gras et de PEG sont de préférence choisis parmi les composés de formule (I) suivante :

R-COO-(CH₂-CH₂-O)ₘH (I)

où R est une chaîne hydrocarbonée linéaire ou ramifiée, saturée ou insaturée, ayant de 10 à 24 atomes de carbone, et m est un nombre entier allant de 8 à 50.

Comme esters d'acides gras et de PEG, on peut citer par exemple les produits d'estérification avec les acides laurique, palmitique, stéarique ou béhénique, et leurs mélanges et avec l'oxyde d'éthylène, notamment ceux comportant de 9 à 50 groupes oxyéthylénés tels que les laurates de PEG-9 à PEG-50 (selon la dénomination CTFA : PEG-9 laurate à PEG-50 laurate) ; les palmitates de PEG-9 à PEG-50 (selon la dénomination CTFA : PEG-9 palmitate à PEG-50 palmitate) ; les stéarates de PEG-9 à PEG-50 (selon la dénomination CTFA : PEG-9 stéarate à PEG-50 stearate) ; les palmito-stéarates de PEG-9 à PEG-50 ; les béhénates de PEG-9 à PEG-50 (selon la dénomination CTFA : PEG-9 behenate à PEG-50 behenate) ; et leurs mélanges.

La composition cosmétique ou dermatologique peut aussi comprendre des émulsionnants additionnels. On peut, à titre d'émulsionnants additionnels, utiliser les sels d'acides gras ayant 8 à 30 atomes de carbone, comme par exemple les sels d'acide palmitique, d'acide stéarique, d'acide béhénique ; les esters gras de glycérol, comme par exemple le glycéryl stéarate ; les dérivés oxyéthylénés des sels d'acides gras et des esters gras de glycérol, comportant 2 à 8 groupes oxyde d'éthylène, et leurs mélanges.

De préférence, les émulsionnants non-ioniques oxyéthylénés et/ou glycérolés de HLB allant de 8 à 18 sont choisis parmi les produits d'addition d'oxyde d'éthylène avec l'alcool cétéarylique ou cétylstéarylique (mélange d'alcool cétylique et d'alcool stéarylique), notamment ceux comportant de 9 à 30 groupes oxyéthylénés, les esters de glycérol et d'acide stéarique et l'alcool cétéarylique.

Encore plus préférentiellement, le système émulsionnant de la composition selon l'invention est composé de au moins 95%, plus préférentiellement au moins 98% voir au moins 99% en poids d'émulsionnants choisis parmi les produits d'addition d'oxyde d'éthylène avec l'alcool cétéarylique ou cétylstéarylique comportant de 10 à 20 groupes oxyéthylénés, du Stéarate de glycéryle et de l'alcool cétéarylique, par rapport au poids total du système émulsionnant.

Idéalement le système émulsionnant de la composition selon l'invention est de préférence constitué du ceteareth-20, du ceteareth-12, de l'alcool cétéarylique et du stéarate de glycéryle.

De manière générale, le (ou les) tensioactif(s) non-ionique(s) oxyéthyléné(s) et/ou glycérolé(s) de HLB allant de 8 à 18 sont présents de préférence en une quantité allant de 2 à 15 %, préférentiellement de 3% à 14%, plus préférentiellement de 4% à 12% et encore plus préférentiellement de 5% à 11% en poids par rapport au poids total de la composition.

### Phase aqueuse :

La composition cosmétique ou dermatologique selon l'invention comprend une quantité de phase aqueuse supérieure ou égale à 50 % en poids par rapport au poids total de la composition, de préférence supérieure ou égale à 55% et plus préférentiellement supérieure ou égale à 60%.

Selon un mode de réalisation de l'invention, la composition de l'invention comprend au moins un polyol (ou alcools polyhydriques) qui est généralement présent dans la phase aqueuse. Comme polyols, on peut citer par exemple la glycérine ; les glycols comme le propylène glycol, le butylène glycol, l'isopropylène glycol et les polyéthylènes glycols tels que le PEG-8 ; le sorbitol ; les sucres tels que le glucose, le fructose, le maltose, le lactose, le sucrose ; et leurs mélanges.

La quantité de polyols va généralement de 0,1% à 60 % en poids et mieux de 0,5% à 50 % en poids par rapport au poids total de la phase aqueuse. De manière classique, la phase aqueuse peut comprendre, outre l'eau et le ou les polyols, un ou plusieurs solvants hydrosolubles choisis parmi les alcool(s) inférieur(s) hydrosoluble(s). On entend par alcool inférieur, un alcool comportant de 1 à 8 atomes de carbone. Comme alcools inférieurs, on peut citer par exemple l'éthanol, l'isopropanol, le butanol et leurs mélanges. Lorsqu'ils sont présents dans la composition de l'invention, le ou les alcool(s) inférieur(s) hydrosoluble(s) peuvent être en une quantité allant de 0,01% à 40 % en poids et de préférence de 0,01% à 20 % en poids par rapport au poids total de la phase aqueuse.

### Additifs :

La composition cosmétique ou dermatologique selon l'invention peut aussi contenir tout adjuvant ou additif habituellement utilisé dans les domaines considérés et notamment dans les domaines cosmétique ou dermatologique. Bien entendu, l'homme du métier veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

Parmi les adjuvants classiques susceptibles d'être contenus dans la phase aqueuse et/ou dans la phase huileuse des nano-émulsions conformes à l'invention (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaine, disodium cocoamphodiacetate) ou non ioniques de HLB supérieure à 10 (tels que POE/PPG/POE, Alkylpolyglucoside, polyglycery1-3hydroxylauryl ether) ; les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes ; les polymères hydrophiles ou lipophiles, anioniques, non ioniques, cationiques ou amphotères, épaississants, gélifiants ou dispersants. Les quantités de ces différents adjuvants sont celles classiquement utilisées dans le domaine considéré, et par exemple de 0,01 à 20 % du poids total de la composition.

Comme actifs utilisables dans la composition cosmétique ou dermatologique de l'invention, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs antibactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, la niacine (vit. PP) ; les amincissants tels que la caféine ; les azurants optiques, et tout actif approprié pour le but final de la composition, et leurs mélanges.

La quantité d'actifs dépend du but recherché. Le ou les actifs peuvent être par exemple présents en une concentration allant de 0,001% à 20%, de préférence de 0,01% à 10% et mieux de 0,05% à 5% en poids par rapport au poids total de la composition. Ces quantités sont calculées en sus des quantités de phases huileuse, émulsionnante et aqueuse détaillées ci-dessus.

### Procédé de préparation :

La composition cosmétique ou dermatologique selon l'invention est une nano-émulsion obtenue par la technique de température d'inversion de phase bien décrite dans l'art antérieur. Ce procédé de préparation comporte au moins les étapes consistant à :
- peser tous les constituants de la composition (à l'exception des matières premières thermosensibles),
- mélanger la phase lipophile avec le système émulsionnant,
- chauffer progressivement et séparément la phase aqueuse et la phase lipophile additionnée du système émulsionnant jusqu'à une température T,
- mélanger et homogénéiser la phase aqueuse et la phase lipophile additionnée du système émulsionnant,
- chauffer le mélange formé sous agitation modérée à une température T1 supérieure ou égale la température d'inversion de phase (température de PIT),
- refroidir brutalement sous agitation faible à 100 tr/min.
- Lorsque la température est redescendue en dessous de la zone d'inversion de phase, additionner éventuellement les matières premières thermosensibles.

Lors du mélange initial, l'émulsion est une émulsion huile-dans-l'eau. Lorsque la chauffe est poursuivie l'émulsion s'inverse à la température de PIT. Une émulsion eau-dans-l'huile est ainsi obtenue. Durant le refroidissement, l'émulsion s'inverse de nouveau et redevient une émulsion huile-dans-l'eau lors du passage à la température de PIT. La vitesse de refroidissement va déterminer la taille des gouttelettes. Il faut, de préférence, que le refroidissement soit brutal et sous agitation faible.

Lorsque la température de PIT est atteinte, le mélange devient transparent ou plus transparent. Dans la zone de formation d'une nano-émulsion (mélange translucide), les interactions hydrophiles et hydrophobes sont équilibrées car la tendance du système émulsionnant est de former aussi bien des micelles directes que des micelles inverses.

### Composition PIT :

La composition cosmétique ou dermatologique selon l'invention comprend avantageusement de 5% à 40 % en poids d'une phase lipophile, de 2% à 15 % en poids d'un système émulsionnant et au moins 50 % en poids d'une phase aqueuse tels que décrits ci-dessus par rapport au poids total de la composition.

De préférence la composition selon l'invention comprend de 10% à 35% en poids d'une phase lipophile et de 3% à 14 % en poids d'un système émulsionnant, plus préférentiellement de 15% à 30% en poids d'une phase lipophile et de 4% à 12 % en poids d'un système émulsionnant, encore plus préférentiellement de 20% à 30% en poids d'une phase lipophile et de 5 à 11 % en poids d'un système émulsionnant, et au moins 50% en poids d'une phase aqueuse tels que décrits ci-dessus par rapport au poids total de la composition.

Le rapport en poids phase lipophile / système émulsionnant de la composition selon l'invention est supérieur ou égal à 0,5 de préférence supérieur ou égal à 1,5 et plus préférentiellement supérieur ou égal à 2,5. Préférentiellement le rapport en poids phase lipophile / système émulsionnant de la composition selon l'invention est de 0,5 à 4.

La composition cosmétique ou dermatologique selon l'invention comprend avantageusement de 5% à 40 % en poids d'une phase lipophile, de 2% à 15 % en poids d'un système émulsionnant et au moins 50 % en poids d'une phase aqueuse tels que décrits ci-dessus par rapport au poids total de la composition et le rapport en poids phase lipophile / système émulsionnant de la composition est de 1 à 4.

De préférence la composition selon l'invention comprend de 10% à 35% en poids d'une phase lipophile, de 3% à 14 % en poids d'un système émulsionnant, et au moins 50% en poids d'une phase aqueuse tels que décrits ci-dessus par rapport au poids total de la composition et le rapport en poids phase lipophile / système émulsionnant de la composition est de 1,5 à 4.

De préférence, la taille moyenne des gouttelettes de la nano-émulsion de la composition selon l'invention va de 0,1 à 0,5 µm caractéristique distinctive des nano-émulsions.

La viscosité dynamique de la composition selon l'invention est de préférence inférieure ou égale à 0.5 Pa.s à 25°C mesurée à l'aide d'un appareillage de Brookfield.

La composition cosmétique ou dermatologique selon l'invention se présente sous forme de crème plus ou moins souple ou d'une émulsion vaporisable, elle peut constituer par exemple une composition cosmétique ou dermatologique comme une composition de démaquillage ou de nettoyage de la peau, des lèvres, une composition solaire (protection U.V.), ou après-solaire, une composition pour le massage de la peau, une composition de baume soin douche, une composition anti-transpirante, une composition de masque, une composition de baume réparateur, une composition gommante et/ou exfoliante aussi bien pour le visage que pour les mains (lorsqu'elle contient des particules exfoliantes), une composition de maquillage, une composition pour lingettes ou encore une composition vaporisable.

La composition de l'invention se caractérise avantageusement par le fait qu'elle présente une stabilité d'une durée supérieure ou égale à 4 semaines, avantageusement supérieure ou égale à 6 semaines, la stabilité étant évaluée après un stockage sans agitation à une température de 50°C et correspondant à une résistance au déphasage évaluée visuellement.

### Utilisation de la composition PIT :

L'invention a encore pour objet l'utilisation cosmétique ou dermatologique de la composition telle que définie ci-dessus pour une application topique.

L'invention a aussi pour objet l'utilisation cosmétique ou dermatologique de la composition telle que définie ci-dessus comme produit de soin de la peau, comme produit d'hygiène, comme produit solaire, comme produit de maquillage.

L'invention a encore pour objet un procédé cosmétique ou dermatologique de traitement de la peau, comprenant l'application sur la peau d'une composition telle que définie ci-dessus.

La composition de l'invention peut également être utilisée pour la formulation de compositions cosmétiques ou de compositions dermatologiques comprenant d'autres composants ou d'autres phases que décrit ci-dessus. Il peut s'agir notamment de la formulation de compositions de soin, d'hygiène, de maquillage.

### EXEMPLES

Dans la suite de la présente description, des exemples sont donnés à titre illustratif de la présente invention et ne visent en aucun cas à en limiter la portée.

Différentes formulations de nano-émulsions à base d'huiles hydrocarbonées ont été évaluées.

Les nano-émulsions selon les exemples comprennent une phase aqueuse, une phase lipophile comprenant au moins une huile hydrocarbonée et un système émulsionnant composé d'émulsionnants huile-dans-l'eau (H/E) non-ioniques.

### Système émulsionnant :

2 émulsionnants H/E non ioniques ont été sélectionnés. Les émulsionnants Eumulgin B2 et Emulgade SEV commercialisés par la société BASF sont particulièrement indiqués pour les émulsions formées par la technique de température d'inversion de phase. Il s'agit d'un système d'émulsionnants non-ioniques, où l'Eumulgin B2 est hydrophile et l'Emulgade SEV lipophile.

Les structures chimiques de ces 2 émulsionnants sont indiquées dans le tableau 1:
- Eumulgin B2, INCI: Ceteareth-20,
- Emulgade SEV, INCI: Glyceryl Stearate and Ceteareth-20 and Ceteareth-12 and Cetearyl Alcohol

**Tableau 1**

| Ceteareth-n où 2<n<100, et m=15 ou 17 | Alcool cétéarylique (13<n<15) | Stéarate de glycéryle |
|---|---|---|
| 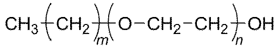 | 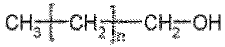 | 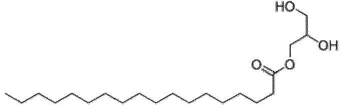 |

### Phase lipophile :

Les différentes phases lipophiles selon les exemples comprennent différentes huiles hydrocarbonées. Les huiles Gemseal 25 ®, Gemseal 60 ® et Gemseal 120 ® commercialisées par la société Total Fluides sont des huiles hydrocarbonées selon l'invention. L'Huile 4 est un exemple comparatif d'huile hydrocarbonée fréquemment retrouvée dans l'état de l'art, l'isohexadécane.

Le Tableau 2 indique les caractéristiques physico-chimiques de chacune des huiles hydrocarbonées selon les exemples.

**Tableau 2**

| **paramètres** | **Gemseal 25 ®** | **Gemseal 60 ®** | **Gemseal 120 ®** | **Huile 4** |
|---|---|---|---|---|
| Distribution en carbones | alcane C13-15 | alcane C18-21 | alcane C21-28 | alcane C16 |
| Gamme de distillation (°C) [ASTM D86] | 220-275 | 220-270 | 330-380 | 210-250 |
| Point d'écoulement (°C) [ASTM D97] | -50 | -0,5 | -30 | -70 |
| Viscosité à 40°C(cSt) [ASTM D445] | 2,5 | 6 | 11 | 3 |
| Aromatiques (%) [spectrometrie UV] | <0,03 | <0,03 | <0,03 | - |
| Mono-aromatiques (ppm) [spectrometrie UV] | 6,5 | 103 | 57 | - |
| Paraffines (%) [GC2D] | 45 | 68 | 46 | 100 |
| Iso-paraffines (%) [GC2D] | < 40 | <60 | <50 | 100 |
| N-paraffines (%) [GC2D] | > 5 | > 8 | >0,01 | 0 |
| Naphtènes (%) [GC2D] | 54,7 | 31,8 | 53,8 | 0 |

Ces huiles Gemseal 25 ®, Gemseal 60 ® et Gemseal 120 ® sont connues pour être non-irritantes, non-occlusives et biodégradables donc compatibles avec une utilisation cosmétique.

De ce fait, les phases lipophiles des nano-émulsions formulées avec les huiles Gemseal 25 ®, Gemseal 60 ® et Gemseal 120 ® sont non toxiques, non irritantes, non comédogènes et biodégradables.

La quantité totale de phase lipophile reste fixe à 25% en poids par rapport au poids total de la nano-émulsion et la quantité d'huile hydrocarbonée comprise dans cette phase lipophile est de 20% en poids par rapport au poids total de la nano-émulsion. Les 5% restants sont compensés pour chaque formulation par un ester classique, plus polaire que l'huile hydrocarbonée paraffinique, l'Isononyl Isononanoate ou ININ, retrouvé très fréquemment dans les émulsions. 25% de phase lipophile représente la quantité de phase lipophile d'un produit pour peau mature, et est donc une quantité significative.

### Phase aqueuse :

La phase aqueuse est majoritairement composée d'eau déminéralisée. Un conservateur est incorporé pour assurer la stabilité microbiologique des formules et de la gomme xanthane est ajoutée pour aider à stabiliser la phase aqueuse de la formule.

### Formulation des nano-émulsions :

Une nano-émulsion est formulée pour chaque huile hydrocarbonée donc 4 nano-émulsions au total sont évaluées et comparées. Les pourcentages d'émulsionnants testés sont ceux donnés dans le tableau 1. Ce sont les doses déterminées à l'aide des doses recommandées par les fournisseurs de ces émulsionnants et du système CAPICO.

Le tableau 3 ci-dessous indique les formulations des 4 nano-émulsions selon les exemples. Les pourcentages indiqués correspondent au poids en matière active par rapport au poids total de la composition.

**Tableau 3**

| | | **Nano-émulsion 1** | **Nano-émulsion 2** | **Nano-émulsion 3** | **Nano-émulsion 4** |
|---|---|---|---|---|---|
| Système émulsionnant | Eumulgin B2 ®+ Emulgade SEV ® | 2,2% + 7,8% | 2,2% + 7,8% | 2,2% + 7,8% | 2,2% + 7,8% |
| Phase lipophile | Gemseal 25 ® | 20,00% | - | - | - |
| | Gemseal 60 ® | - | 20,00% | - | - |
| | Gemseal 120 ® | - | - | 20,00% | - |
| | Huile 4 | - | - | - | 20,00% |
| | Ester (Isononyl Isononanoate) | 5,00% | 5,00% | 5,00% | 5,00% |
| Phase Aqueuse | Eau Déminéralisée | QSP | QSP | QSP | QSP |
| | Gomme xanthane | 0,20% | 0,20% | 0,20% | 0,20% |
| | Conservateur | 0,50% | 0,50% | 0,50% | 0,50% |

Les phases aqueuse et lipophile sont chauffées à 60-65°C séparément, puis la phase lipophile est versée dans la phase aqueuse ; l'émulsion ainsi formée est chauffée à 80°C sous agitation modérée, puis refroidie brutalement sans agitation.

### Résultats :

300g de nano-émulsions sont formulés pour chacun des exemples. Chacune des nano-émulsions est ensuite conditionnée dans un pot de 200g et dans un pot de 30g.

### Température d'inversion de phase :

La température de PIT est mesurée au moment de la formulation de chaque nano-émulsion.

Le tableau 4 regroupe les mesures de températures de PIT pour chacune des 4 nano-émulsions.

**Tableau 4**

| | **Nano-émulsion 1** | **Nano-émulsion 2** | **Nano-émulsion 3** | **Nano-émulsion 4** |
|---|---|---|---|---|
| Huile hydrocarbonée | Gemseal 25 ® | Gemseal 60 ® | Gemseal 120 ® | Huile 4 |
| Température PIT (en °C) | 53 | 57 | 62 | 55 |

Pour chacune des formulations des nano-émulsions 1 à 3 selon les exemples, on obtient des températures de PIT basses et intéressantes au niveau industriel.

Ces températures d'inversion de phase très basses permettent d'éviter les problèmes liés à une chauffe importante : dénaturation des composés, dépenses énergétiques.

### Stabilité :

La stabilité des nano-émulsions selon les exemples est évaluée de façon visuelle à une température de 50°C. Cette observation au bout d'1 semaine et de 4 semaines permet de voir si un déphasage de la composition de chaque nano-émulsion est apparu.

Le tableau 5 indique si une séparation des différentes phases constitutives de chaque nano-émulsion est constatée ou non.

**Tableau 5**

| | | **Nano-émulsion 1** | **Nano-émulsion 2** | **Nano-émulsion 3** | **Nano-émulsion 4** |
|---|---|---|---|---|---|
| | Huile hydrocarbonée | Gemseal 25 ® | Gemseal 60 ® | Gemseal 120 ® | Huile 4 |
| Déphasage | 1 semaine | Non | Non | Non | Oui |
| | 4 semaines | Non | Non | Non | Oui |

Les résultats des différents tests de stabilité démontrent que les nano-émulsions 1 à 3 sont totalement stables au cours du temps contrairement à la nano-émulsion 4 pour laquelle un déphasage de la composition est observé dès la première semaine.

Les différentes formulations de nano-émulsions 1, 2 et 3 selon l'invention n'ont pas révélé d'incompatibilité entre les huiles hydrocarbonées exemplifiées et le système PIT. Au contraire, ces huiles hydrocarbonées permettent d'obtenir, comme le démontrent les différents résultats, des nano-émulsions stables, peu visqueuses, non-irritantes, non-occlusives avec une température de PIT faible.

## Revendications

1. Composition cosmétique ou dermatologique sous forme d'une nano-émulsion huile-dans-eau **caractérisée en ce qu'**elle comprend :
- une phase lipophile (a) qui comprend au moins une huile hydrocarbonée comprenant au moins une isoparaffine, et au moins un cyclo-alcane,
- un système émulsionnant (b) comprenant au moins 95% en poids d'émulsionnants choisis parmi les produits d'addition d'oxyde d'éthylène avec l'alcool cétéarylique ou cétylstéarylique comportant de 10 à 20 groupes oxyéthylénés, du Stéarate de glycéryle et de l'alcool cétéarylique, par rapport au poids total du système émulsionnant,
- une phase aqueuse (c),
- le rapport en poids phase lipophile (a) / système émulsionnant (b) étant supérieur ou égal à 0,5.

2. Composition selon la revendication 1, **caractérisée en ce que** la quantité de phase lipophile (a) est de 5% à 40%, préférentiellement de 10% à 35%, plus préférentiellement de 15% à 30% et encore plus préférentiellement de 20% à 30% en poids par rapport au poids total de la composition.

3. Composition selon l'une quelconque des revendications précédentes, **caractérisée en ce que** la phase lipophile (a) comprend de 15% à 100% en poids d'huile hydrocarbonée, préférentiellement de 20% à 90%, plus préférentiellement de 25% à 80% et encore plus préférentiellement de 30% à 70% en poids d'huile hydrocarbonée par rapport au poids total de la phase lipophile.

4. Composition selon l'une quelconque des revendications précédentes, **caractérisée en ce que** l'huile hydrocarbonée est une huile hydrocarbonée hydrogénée.

5. Composition selon l'une quelconque des revendications précédentes, **caractérisée en ce que** l'huile hydrocarbonée est choisie parmi les isoparaffines, les paraffines normales et les cyclo alcanes comprenant 12 à 29 atomes de carbone, préférentiellement 13 à 23 atomes de carbone et plus préférentiellement 18 à 21 atomes de carbone.

6. Composition selon l'une quelconque des revendications précédentes, **caractérisée en ce que** l'huile hydrocarbonée a une teneur en composés paraffiniques allant de 40% à 99%, préférentiellement de 45% à 90% et plus préférentiellement de 60% à 80%.

7. Composition selon l'une quelconque des revendications précédentes, **caractérisée en ce que** l'huile hydrocarbonée a une teneur en composés naphténiques allant de 1% à 60%, préférentiellement de 10% à 55 % et plus préférentiellement de 20% à 40 %.

8. Composition selon l'une quelconque des revendications précédentes, **caractérisée en ce que** l'huile hydrocarbonée a une teneur en isoparaffines allant de 30% à 90%, de préférence de 40% à 80% et plus préférentiellement de 50% à 70%.

9. Composition selon l'une quelconque des revendications précédentes, **caractérisée en ce que** l'huile hydrocarbonée a une teneur en paraffines normales allant de 0,01% à 30%, de préférence de 0,01% à 25% et plus préférentiellement de 5% à 20%.

10. Composition selon l'une quelconque des revendications précédentes, **caractérisée en ce que** l'huile hydrocarbonée comprend de 30% à 90% d'isoparaffines, de 0,01% à 30% de paraffines normales et de 1% à 60% de composés naphténiques.

11. Composition selon l'une quelconque des revendications précédentes, **caractérisée en ce que** la quantité de système émulsionnant (b) est de 2% à 15%, préférentiellement de 3% à 14%, plus préférentiellement de 4% à 12% et encore plus préférentiellement de 5% à 11% en poids par rapport au poids total de la composition.

12. Composition selon l'une quelconque des revendications précédentes, **caractérisée en ce que** le système émulsionnant (b) comprend au moins un émulsionnant lipophile et un émulsionnant hydrophile.

13. Composition selon l'une quelconque des revendications précédentes, **caractérisée en ce que** le système émulsionnant est constitué de ceteareth-20, du ceteareth-12, de l'alcool cétéarylique et du stéarate de glycéryle.

14. Composition selon l'une quelconque des revendications précédentes, **caractérisée en ce qu'**elle est préparée par la technique de température d'inversion de phase.

15. Utilisation cosmétique de la composition selon l'une quelconque des revendications précédentes pour une application topique, notamment comme produit de soin solaire, comme produit de soin de la peau, comme produit de maquillage.

16. Utilisation de la composition selon l'une quelconque des revendications 1 à 14 pour la formulation de produits cosmétiques ou de produits dermatologiques.

## Patentansprüche

1. Kosmetische oder dermatologische Zusammensetzung in Form einer Öl-in-Wasser-Nanoemulsion, **dadurch gekennzeichnet, dass** sie Folgendes umfasst:
- eine lipophile Phase (a), die mindestens ein Kohlenwasserstofföl, das mindestens ein Isoparaffin und mindestens ein Cycloalkan umfasst, umfasst,
- ein Emulgiersystem (b), umfassend mindestens 95 Gew.-% Emulgatoren, die aus Additionsprodukten von Ethylenoxid mit Cetearylalkohol oder Cetylstearylalkohol mit 10 bis 20 Oxyethylengruppen, Glycerylstearat und Cetearylalkohol ausgewählt sind, bezogen auf das Gesamtgewicht des Emulgiersystems,
- eine wässrige Phase (c),
- wobei das Gewichtsverhältnis von lipophiler Phase (a) zu Emulgiersystem (b) größer oder gleich 0,5 ist.

2. Zusammensetzung nach Anspruch 1, **dadurch gekennzeichnet, dass** die Menge der lipophilen Phase (a) 5 bis 40 Gew.-%, bevorzugt 10 bis 35 Gew.-%, weiter bevorzugt 15 bis 30 Gew.-% und noch weiter bevorzugt 20 bis 30 Gew.-%, bezogen auf das Gesamtgewicht der Zusammensetzung, beträgt.

3. Zusammensetzung nach einem der vorhergehenden Ansprüche, **dadurch gekennzeichnet, dass** die lipophile Phase (a) 15 bis 100 Gew.-% Kohlenwasserstofföl, bevorzugt 20 bis 90 Gew.-%, weiter bevorzugt 25 bis 80 Gew.-% und noch weiter bevorzugt 30 bis 70 Gew.-% Kohlenwasserstofföl, bezogen auf das Gesamtgewicht der lipophilen Phase, umfasst.

4. Zusammensetzung nach einem der vorhergehenden Ansprüche, **dadurch gekennzeichnet, dass** es sich bei dem Kohlenwasserstofföl um ein hydriertes Kohlenwasserstofföl handelt.

5. Zusammensetzung nach einem der vorhergehenden Ansprüche, **dadurch gekennzeichnet, dass** das Kohlenwasserstofföl aus Isoparaffinen, Normalparaffinen und Cycloalkanen mit 12 bis 29 Kohlenstoffatomen, bevorzugt 13 bis 23 Kohlenstoffatomen und weiter bevorzugt 18 bis 21 Kohlenstoffatomen, ausgewählt ist.

6. Zusammensetzung nach einem der vorhergehenden Ansprüche, **dadurch gekennzeichnet, dass** das Kohlenwasserstofföl einen Gehalt an paraffinischen Verbindungen im Bereich von 40 bis 99 %, bevorzugt von 45 bis 90 % und weiter bevorzugt von 60 bis 80 % aufweist.

7. Zusammensetzung nach einem der vorhergehenden Ansprüche, **dadurch gekennzeichnet, dass** das Kohlenwasserstofföl einen Gehalt an napthenischen Verbindungen im Bereich von 1 bis 60 %, bevorzugt 10 bis 55 % und noch weiter bevorzugt 20 bis 40 % aufweist.

8. Zusammensetzung nach einem der vorhergehenden Ansprüche, **dadurch gekennzeichnet, dass** das Kohlenwasserstofföl einen Gehalt an Isoparaffinen im Bereich von 30 bis 90 %, bevorzugt von 40 bis 80 % und weiter bevorzugt von 50 bis 70 % aufweist.

9. Zusammensetzung nach einem der vorhergehenden Ansprüche, **dadurch gekennzeichnet, dass** das Kohlenwasserstofföl einen Gehalt an Normalparaffinen im Bereich von 0,01 bis 30 %, bevorzugt von 0,01 bis 25 % und weiter bevorzugt von 5 bis 20 % aufweist.

10. Zusammensetzung nach einem der vorhergehenden Ansprüche, **dadurch gekennzeichnet, dass** das Kohlenwasserstofföl 30 bis 90 % Isoparaffine, 0,01 bis 30 % Normalparaffine und 1 bis 60 % naphthenische Verbindungen umfasst.

11. Zusammensetzung nach einem der vorhergehenden Ansprüche, **dadurch gekennzeichnet, dass** die Menge des Emulgiersystems (b) 2 bis 15 Gew.-%, bevorzugt 3 bis 14 Gew.-%, weiter bevorzugt 4 bis 12 Gew.-% und noch weiter bevorzugt 5 bis 11 Gew.-%, bezogen auf das Gesamtgewicht der Zusammensetzung, beträgt.

12. Zusammensetzung nach einem der vorhergehenden Ansprüche, **dadurch gekennzeichnet, dass** das Emulgiersystem (b) mindestens einen lipophilen Emulgator und einen hydrophilen Emulgator umfasst.

13. Zusammensetzung nach einem der vorhergehenden Ansprüche, **dadurch gekennzeichnet, dass** das Emulgiersystem aus Ceteareth-20, Ceteareth-12, Cetearylalkohol und Glycerylstearat besteht.

14. Zusammensetzung nach einem der vorhergehenden Ansprüche, **dadurch gekennzeichnet, dass** sie durch die Phaseninversionstemperaturtechnik hergestellt wird.

15. Kosmetische Verwendung der Zusammensetzung nach einem der vorhergehenden Ansprüche zur topischen Anwendung, insbesondere als Sonnenschutzprodukt, als Hautpflegeprodukt oder als Make-up-Produkt.

16. Verwendung der Zusammensetzung nach einem der Ansprüche 1 bis 14 zur Formulierung von kosmetischen Produkten oder dermatologischen Produkten.

## Claims

1. Cosmetic or dermatological composition in the form of an oil-in-water nanoemulsion, **characterized in that** it comprises:
- a lipophilic phase (a) which comprises at least one hydrocarbon-based oil comprising at least one isoparaffin, and at least one cycloalkane,
- an emulsifying system (b) comprising at least 95% by weight of emulsifiers chosen from adducts of ethylene oxide with cetearyl or cetylstearyl alcohol comprising from 10 to 20 oxyethylene groups, glyceryl stearate and cetearyl alcohol, relative to the total weight of the emulsifying system,
- an aqueous phase (c),
- the lipophilic phase (a)/emulsifying system (b) weight ratio being greater than or equal to 0.5.

2. Composition according to Claim 1, **characterized in that** the amount of lipophilic phase (a) is from 5% to 40%, preferentially from 10% to 35%, more preferentially from 15% to 30% and even more preferentially from 20% to 30% by weight relative to the total weight of the composition.

3. Composition according to either of the preceding claims, **characterized in that** the lipophilic phase (a) comprises from 15% to 100% by weight of hydrocarbon-based oil, preferentially from 20% to 90%, more preferentially from 25% to 80% and even more preferentially from 30% to 70% by weight of hydrocarbon-based oil relative to the total weight of the lipophilic phase.

4. Composition according to any one of the preceding claims, **characterized in that** the hydrocarbon-based oil is a hydrogenated hydrocarbon-based oil.

5. Composition according to any one of the preceding claims, **characterized in that** the hydrocarbon-based oil is chosen from isoparaffins, normal paraffins and cycloalkanes comprising 12 to 29 carbon atoms, preferentially 13 to 23 carbon atoms and more preferentially 18 to 21 carbon atoms.

6. Composition according to any one of the preceding claims, **characterized in that** the hydrocarbon-based oil has a content of paraffinic compounds ranging from 40% to 99%, preferentially from 45% to 90% and more preferentially from 60% to 80%.

7. Composition according to any one of the preceding claims, **characterized in that** the hydrocarbon-based oil has a content of naphthenic compounds ranging from 1% to 60%, preferentially from 10% to 55% and more preferentially from 20% to 40%.

8. Composition according to any one of the preceding claims, **characterized in that** the hydrocarbon-based oil has a content of isoparaffins ranging from 30% to 90%, preferably from 40% to 80% and more preferentially from 50% to 70%.

9. Composition according to any one of the preceding claims, **characterized in that** the hydrocarbon-based oil has a content of normal paraffins ranging from 0.01% to 30%, preferably from 0.01% to 25% and more preferentially from 5% to 20%.

10. Composition according to any one of the preceding claims, **characterized in that** the hydrocarbon-based oil comprises from 30% to 90% of isoparaffins, from 0.01% to 30% of normal paraffins and from 1% to 60% of naphthenic compounds.

11. Composition according to any one of the preceding claims, **characterized in that** the amount of emulsifying system (b) is from 2% to 15%, preferentially from 3% to 14%, more preferentially from 4% to 12% and even more preferentially from 5% to 11 % by weight relative to the total weight of the composition.

12. Composition according to any one of the preceding claims, **characterized in that** the emulsifying system (b) comprises at least one lipophilic emulsifier and one hydrophilic emulsifier.

13. Composition according to any one of the preceding claims, **characterized in that** the emulsifying system is constituted of Ceteareth-20, Ceteareth-12, cetearyl alcohol and glyceryl stearate.

14. Composition according to any one of the preceding claims, **characterized in that** it is prepared via the phase inversion temperature technique.

15. Cosmetic use of the composition according to any one of the preceding claims for topical application, especially as a suncare product, as a skincare product or as a makeup product.

16. Use of the composition according to any one of Claims 1 to 14 for the formulation of cosmetic products or dermatological products.
